# EUROPEAN PATENT SPECIFICATION

(11) **EP 3 450 426 B1**
(45) Date of publication and mention of the grant of the patent: **01.09.2021**
(21) Application number: 18798245.9
(22) Date of filing: 08.05.2018
(51) Int. Cl.: C07D 209/08

(54) **METHOD FOR SYNTHESIZING SILODOSIN AND INTERMEDIATE THEREOF**
VERFAHREN ZUR SYNTHESE VON SILODOSIN UND ZWISCHENPRODUKT DARAUS
PROCÉDÉ POUR LA SYNTHÈSE DE SILODOSINE ET D'UN INTERMÉDIAIRE DE CELLE-CI

(30) Priority: 10.05.2017 CN 201710324907
(43) Date of publication of application: 06.03.2019
(73) Proprietor: Zhejiang Tianyu Pharmaceutical Co., Ltd., Huangyan Taizhou Zhejiang 318020 (CN)
(72) Inventor: ZHU, Guorong, Taizhou Zhejiang 318020 (CN); WANG, Zhen, Taizhou Zhejiang 318020 (CN); YANG, Huilin, Taizhou Zhejiang 318020 (CN); HUANG, Rongming, Taizhou Zhejiang 318020 (CN); TU, Yongjun, Taizhou Zhejiang 318020 (CN)
(74) Representative: Dehns
(86) International application number: PCT/CN2018/085967
(87) International publication number: WO 2018/205919

(56) References cited:
- WO-A1-2012/147019
- WO-A1-2015/126076
- WO-A1-2015/126076
- WO-A1-2017/051324
- WO-A1-2017/055664
- WO-A2-2012/131710
- CN-A- 104 974 072
- CN-A- 106 045 895
- CN-A- 107 056 675
- JP-A- 2016 003 183
- KR-A- 20150 066 777
- KR-A- 20150 066 777
- KR-A- 20160 027 536
- KR-A- 20160 027 537
- FEI ZHAO ET AL: "Design, Synthesis, and Biological Evaluation of Indoline and Indole Derivatives as Potent and Selective [alpha] 1A -Adrenoceptor Antagonists", JOURNAL OF MEDICINAL CHEMISTRY, vol. 59, no. 8, 13 April 2016 (2016-04-13) , pages 3826-3839, XP055621145, US ISSN: 0022-2623, DOI: 10.1021/acs.jmedchem.5b02023

## Description

### TECHNICAL FIELD

The present invention relates to the field of medicinal chemistry, in particular to a method for synthesizing silodosin and an intermediate thereof.

### TECHNICAL BACKGROUND

Silodosin is an α-adrenergic receptor antagonist invented by Kissei Pharmaceutical Co., Ltd. in Japan, which is clinically used for urinary dysfunction associated with benign prostatic hyperplasia. The chemical name of silodosin is: 2,3-dihydro-l-(3-hydroxypropyl)-5-[(2R)-2-[2-[2-(2,2,2-trifluoroethoxy)phenoxy]ethylamino]pro pyl]-1H-indole-7-carboxamide, the chemical structure of which is shown in formula 1.

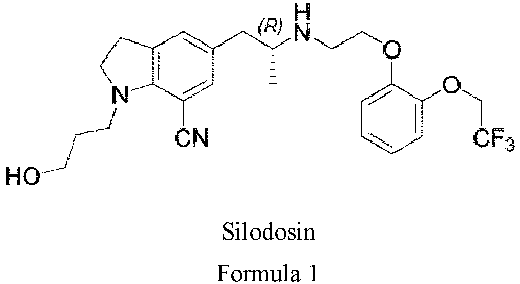

Patent US 5,387, 603 discloses a silodosin compound and its basic synthesis route (formula 2). After indoline is protected by acetyl group, Friedel-Crafts acylation is carried out between the protected indoline and propionyl chloride, then halogenations is carried out with concentrated sulfuric acid/hydrobromic acid to obtain halogenated ketone. The carbonyl of the halogenated ketone is reduced by triethylsilane/trifluoroacetic acid system, and the obtained halogenated substance is sequentially subjected to nitration, reduction, Sandmeyer reaction to introduce a cyano group at the 7-position of the indoline, the compound comprising the cyano group reacts with sodium azide, then the product is subjected to reduction by palladium/barium sulfate and chiral resolution to obtain propylamine in the R configuration, followed by the condensation with 2-[2-(2,2,2-trifluoroethoxy)phenoxy]ethyl methanesulfonate. The amino of the obtained product is protected with Boc, then the protected product is subjected to deacetylation, oxidation hydrolysis, benzoyloxypropyl connection, benzoyl deprotection and Boc deprotection to obtain silodosin.

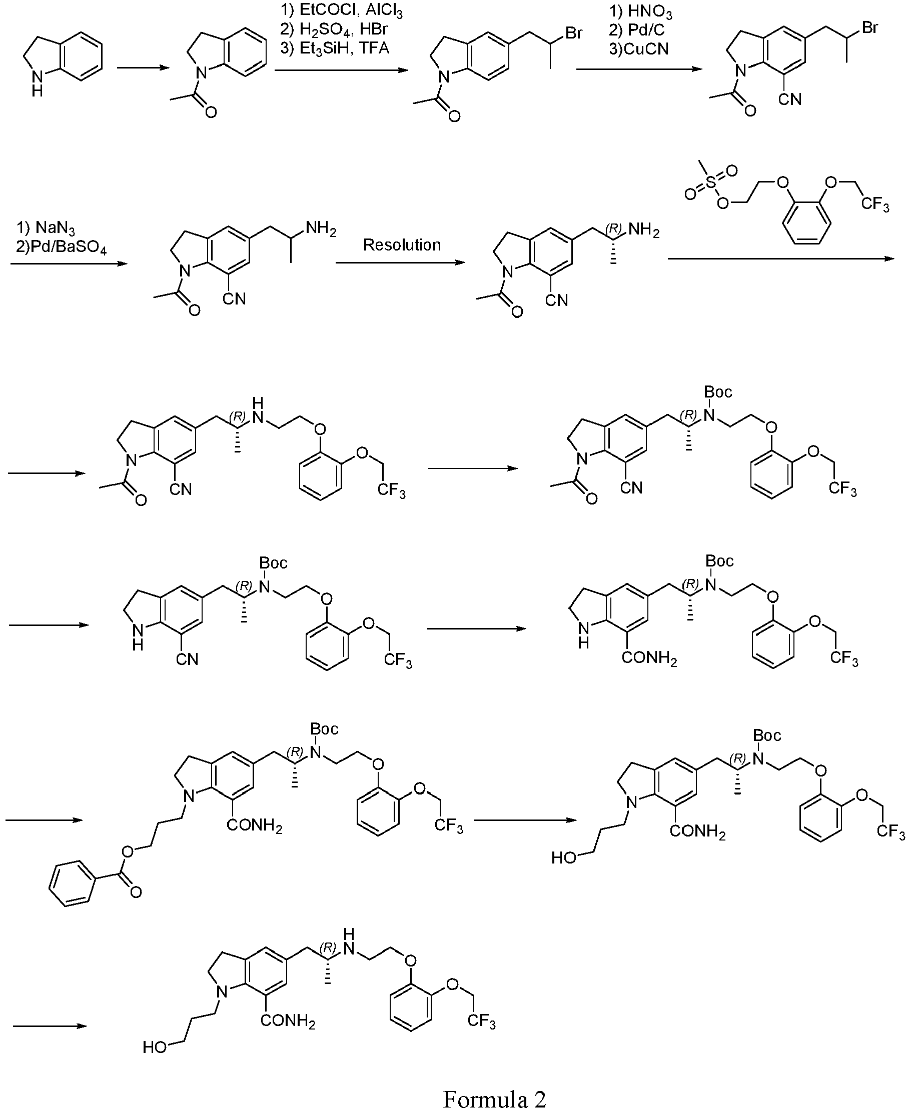

In summary, the existing synthetic process has deficiencies such as lengthy steps, a large number of protection and deprotection steps, a low total yield, the requirement of dangerous reactions such as nitrification, azidation, cyanation, and it is hard to control the process conditions. It is necessary to improve the synthetic process of silodosin and its intermediates, increase yields, and reduce production costs and safety risks.

CN 104974072 discloses a preparation method of a silodosin intermediate which employs indoline as a starting material.

KR 20150066777 discloses a new indoline derivative for use as a synthetic intermediate of silodosin, and a preparation method thereof.

FEI ZHAO ET AL, "Design, Synthesis, and Biological Evaluation of Indoline and Indole Derivatives as Potent and Selective [alpha] 1A-Adrenoceptor Antagonists", JOURNAL OF MEDICINAL CHEMISTRY, 2016, Vol. 59, No. 8, pages 3826-3839 discloses the synthesis of a series of indoline and indole derivatives.

WO 2012/147019 discloses the preparation of a tartrate salt of 3-[7-cyano-5[(2R)-2-({2-[2-(2,2,2-trifluoroethoxy)phenoxy]ethyl}amino)propyl]-2,3-dihydro-1H-indol-1-yl}propyl benzoate, which is a precursor in the preparation of silodosin.

WO 2017/055664 discloses a maleic acid salt of an intermediate used in the manufacture of silodosin, and the process of preparing the salt and its use in the preparation of silodosin.

WO 2015/126076 discloses 3-{7-cyano-5-[(2R)-2-({2-[2-(2,2,2-trifluoroethoxy)phenoxy] ethyl}amino)propyl]-2,3-dihydro-1H-indol-1-yl}propyl benzoate 2-oxoglutarate as an intermediate used in the preparation of silodosin.

KR 20160027537 discloses a method of preparing silodosin.

KR 20160027536 discloses a method preparing of 3-{7-cyano-5[(2R)-2-({2-[2-(2,2,2-trifluoroethoxy)phenoxy]ethyl}aminopropyl)-2,3-dihydro-1H-indole-1-yl} propylene benzoate, which is an intermediate in the synthesis of silodosin.

### SUMMARY OF THE INVENTION

The object of the present invention is to overcome the above deficiencies in the prior art, and to provide a novel method for synthesizing silodosin and an intermediate thereof, and the synthetic method of the present invention improves the synthetic process of silodosin and its intermediates, avoiding the use of the dangerous reagent sodium azide, reducing production risk and increasing production yield.

Specifically, a method for synthesizing a silodosin intermediate 10 is carried out by the following reaction steps: 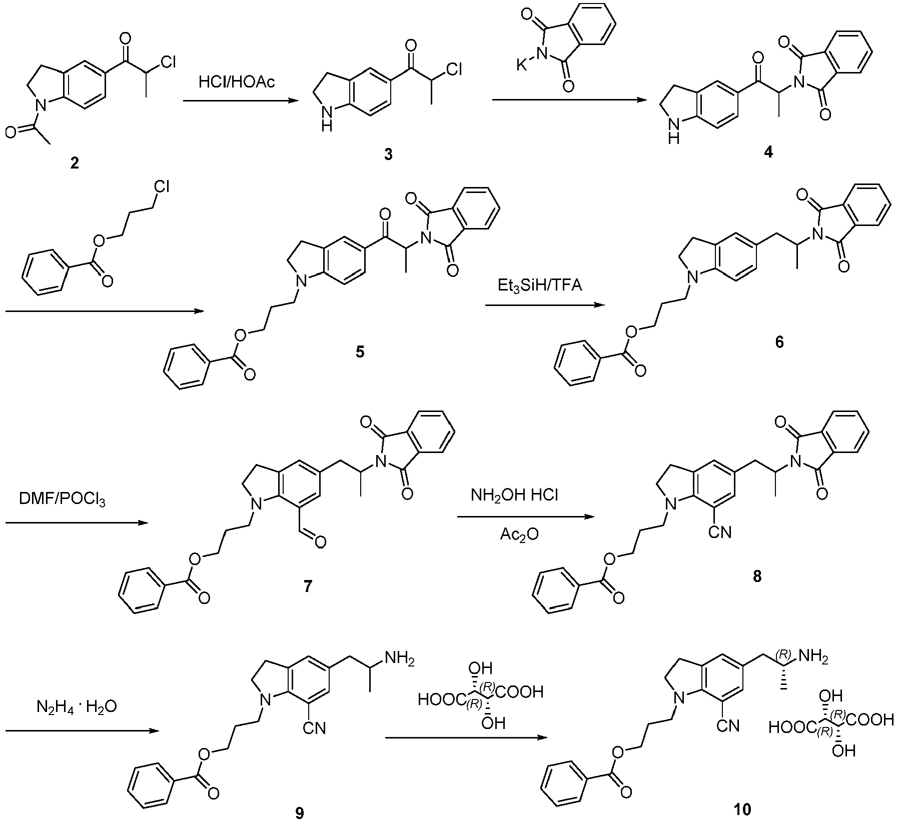 the specific steps are:
step 1, chloride 2 is deacetylated with hydrochloric acid and acetic acid to obtain indoline 3,
step 2, indoline 3 and potassium phthalimide are subjected to SN2 substitution reaction in an organic solvent under the presence of potassium carbonate to obtain imide 4.
step 3, N-alkylation reaction is carried out between imide 4 and 3-chloropropyl benzoate in an organic solvent at a certain temperature under the presence of a phase transfer catalyst, potassium carbonate and potassium iodide to obtain benzoate 5,
step 4, the carbonyl of benzoate 5 is reduced by triethylsilane and trifluoroacetic acid to obtain indoline 6,
step 5, the indoline 6 is subjected to Vilsmeier reaction under the action of dimethylformamide and phosphorus oxychloride to obtain aldehyde 7,
step 6, aldehyde 7 is subjected to oximation and dehydration to obtain nitrile 8,
step 7, nitrile 8 reacts with hydrazine hydrate under the protection of inert gas to remove the phthaloyl group, and the obtained amine 9 is resolved by L-tartaric acid to obtain silodosin intermediate 10.

Preferably, in step 1, the rate of charge (by mass) of compound 2, hydrochloric acid and acetic acid is 1:(5-10):(5-10).

Preferably, in step 2, the molar ratio of compound 3, potassium phthalimide and potassium carbonate is 1:(1.0-2.0):(1.0-2.0).

Preferably, in step 2, the molar ratio of compound 3, potassium phthalimide and potassium carbonate is 1:1.05:1.1.

Preferably, in step 2, the organic solvent can be selected from acetonitrile, toluene, tetrahydrofuran etc., more preferably, the organic solvent is acetonitrile.

Preferably, in step 3, the phase transfer catalyst is selected from tetrabutylammonium bromide, tetrabutylammonium chloride, tetrabutylammonium iodide, benzyltrimethylammonium chloride, benzyltriethylammonium chloride..

Preferably, in step 3, the molar ratio of compound 4 to 3-chloropropyl benzoate, potassium carbonate, potassium iodide, and the phase transfer catalyst is 1 :(1.0-2.0):(2.0-4.0):(1.0-2.0):(0.05-0.1).

Preferably, in step 3, the molar ratio of compound 4 to 3-chloropropyl benzoate, potassium carbonate, potassium iodide, and the phase transfer catalyst is 1:1.6:2.4:1.6:0.07.

Preferably, in step 3, the reaction temperature is 90-100 °C.

Preferably, in step 3, the organic solvent is selected from dimethylformamide, dimethylacetamide, dimethyl sulfoxide, N-methyl pyrrolidone, more preferably, the organice solvent is dimethylformamide.

Preferably, in step 4, the molar ratio of compound 5, triethylsilane and trifluoroacetic acid is 1:(2.0-3.0):(2.0-3.0), preferably 1:2.0:2.6. The organic solvent of step 4 can be selected from dichloromethane, tetrahydrofuran.

Preferably, in step 5, the molar ratio of compound 6 and phosphorus oxychloride is 1:(2.0-3.0), preferably 1:2.0.

Preferably, in step 6, the molar ratio of compound 7, hydroxylamine hydrochloride and potassium carbonate is 1:(1.0-2.0):(1.0-2.0), preferably 1:1.0:1.0.

The present invention also discloses a method for synthesizing silodosin according to claim 9. Specifically, it discloses a method for synthesizing silodosin which comprises the step of preparing the above intermediate 10 as recited herein, and synthesizing silodosin from said intermediate 10 according to the following reaction steps: 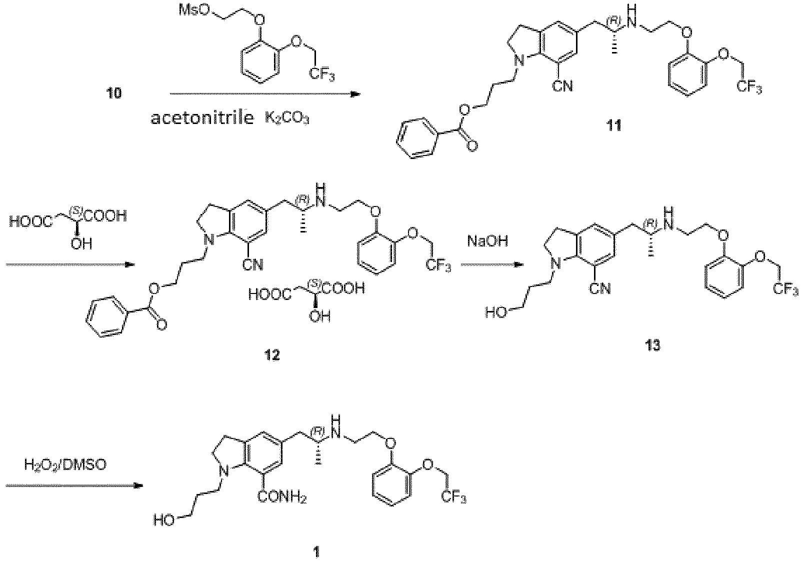 the specific steps are:
step 8, in the organic solvent acetonitrile, a condensation reaction is carried out between the intermediate 10 of silodosin and 2-[2-(2,2,2-trifluoroethoxy)phenoxy]ethyl methanesulfonate under the presence of potassium carbonate to give compound 11; preferably, the molar ratio of compound 10, 2-[2-(2,2,2-trifluoroethoxy)phenoxy]ethyl methanesulfonate and potassium carbonate is 1:(1.0-1.5):(1.5-2.5), more preferably 1:1.0:2.0;
step 9, in the mixed solvent of ethyl acetate/n-hexane, the obtained compound 11 and L-malic acid form salt 12 by crystallization. Preferably, the molar ratio of compound 11 and malic acid is 1:(1.0-1.1), more preferably 1:1.05. Preferably, the volume ratio of ethyl acetate and n-hexane is 1: 1;
step 10, under alkaline condition, the benzoyl protection of compound 12 is removed to give nitrile 13;
step 11, in the organic solvent DMSO, under alkaline condition provided by potassium carbonate, compound 12 is hydrolyzed by hydrogen peroxide to obtain silodosin 1, preferably, the molar ratio of compound 13 and hydrogen peroxide is 1:(2.0-5.0), more preferably 1:2.0.
Salt 12 produced by the above step 9 is crystal form I, 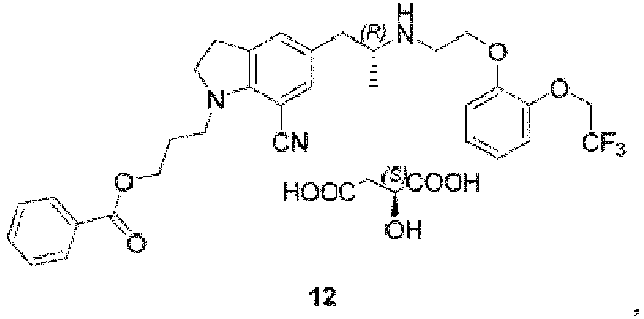 crystal form I of salt 12 has a melting point of 71-73°C, a peak is found at 71.82 ° C in the DSC thermogram of crystal form I of salt 12. The powder X-ray diffraction pattern of crystal form I of salt 12 has the following characteristic peaks represented by 2θ angles: 8.6°±0.2°, 11.3°±0.2°, 11.4°±0.2°, 11.8°±0.2°, 14.2°±0.2°, 15.3°±0.2°, 17.6°±0.2°, 20.1°±0.2°, 22.8°±0.2°, 23.7°±0.2°, 24.0°±0.2°, 24.6°±0.2°, 34.6°±0.2°, 35.8°±0.2°.

In the present application, the room temperature represents 25 °C, the cold saturated sodium carbonate aqueous solution represents saturated sodium carbonate aqueous solution at 5 °C, the rate of the change of the temperature when the temperature is slowly decreased or when the temperature is slowly increased is 10 °C per hour.

The beneficial effects of the present invention are:
(1) the invention simplifies the conversion step, the reaction yield is high in each step, which effectively reduces the industrial production cost and risk, and the invention is suitable for industrial production.
(2) compound 11 and L-malic acid form a salt by crystallization in the present invention, which effectively removes related impurities which are difficult to be removed, such as dimer, and is beneficial to synthesize high-quality silodosin in the subsequent steps, thereby improving the yield.

### BRIEF DESCRIPTION OF THE DRAWINGS

Figure 1 is an X-ray powder diffraction pattern of crystal form I of compound 12 prepared in the examples of the present application.
Figure 2 is a DSC thermogram of crystal form I of compound 12 prepared in the examples of the present application.

### EMBODIMENTS

The present invention will be further described in the following specific embodiments in order to provide a better understanding of the invention. But the present invention is not limited to the scope of the examples. The experiment methods which do not specify the conditions in the following examples are selected according to conventional methods and conditions, or according to the product specifications.

Compound 2 is prepared according to the method disclosed in patent CN102690223, the other solvents and reagents are all bought from Nanjing Chemlin Chemical Industry Co., Ltd.

¹HNMR is measured by Brukeravance 400 nuclear magnetic resonance instrument; HRMS is measured by Waters Xevo G2-XS QTof high resolution mass spectrometer, the ion source is ESI source; the melting point is measured by Optimelt MPA100 full-automatic melting point meter; HPLC is measured by Agilent 1260 high performance liquid chromatograph.

### Example 1: Preparation of Compound 3

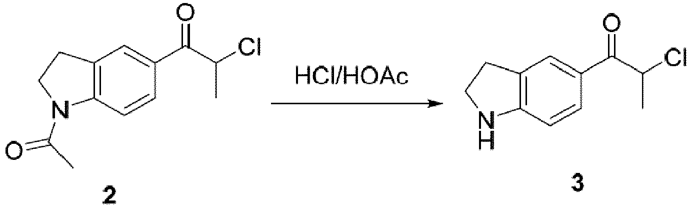

At room temperature, 251 g of compound 2, 1500 mL of 30% hydrochloric acid and 1000 mL of acetic acid were fed into the reaction vessel; then the temperature was slowly increased to 80-90 °C to carry out the reaction, liquid chromatograph was used to monitor the reaction until it was completed (4-10 hours), the reaction solution was concentrated to dryness under reduced pressure, 1500 mL of dichloromethane and 2000 mL of cold saturated sodium carbonate aqueous solution were added, and the mixture layered after being stirred for 1 hour. The organic layer was washed with saturated salt solution, the organic layer was collected then was dried over anhydrous sodium sulfate, then was filtered, and the filtrate was concentrated to dryness under reduced pressure to obtain 203 g of yellow solid compound 3, yield: 97%. Melting point: 91-93 °C; HRMS m/z (ESI): C₁₁H₁₃ClNO [M+H⁺] theoretical value: 210.0680, measured value: 210.0689; ¹ H-NMR (400 MHz, CDCl₃) δ: 7.74 (m, 2H), 6.55 (d, *J* = 8.4 Hz, 1H), 5.22 (q, *J* = 6.4 Hz, 1H), 4.38 (br, 1H), 2.69 (t, *J* = 8.4 Hz, 2H), 3.09 (t, *J* = 8.4 Hz, 2H), 1.71 (d, *J* = 6.4 Hz, 3H). ¹³C-NMR (100 MHz, CDCl₃) δ: 191.73, 156.82, 131.14, 129.13, 125.86, 124.17, 107.06, 52.82, 47.18, 28.63, 20.50.

### Example 2: Preparation of Compound 4

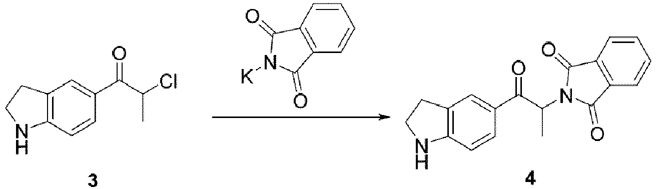

At room temperature, 209 g of compound 3 obtained from example 1, 194 g of potassium phthalimide, 1000 mL of acetonitrile and 151 g of potassium carbonate were fed into the reaction vessel, wherein the molar ratio of compound 3, potassium phthalimide and potassium carbonate was 1:1.05:1.1.

Then the temperature was slowly increased to 80-90 °C to carry out the reaction, liquid chromatograph was used to monitor the reaction until it was completed (4-10 hours), the reaction solution was concentrated to dryness under reduced pressure, 1500 mL of dichloromethane and 2000 mL of cold saturated sodium carbonate aqueous solution were added, and the mixture layered after being stirred for 1 hour. The organic layer was washed with saturated salt solution, the organic layer was collected then was dried over anhydrous sodium sulfate, then was filtered, and the filtrate was concentrated to dryness under reduced pressure to obtain 295 g of yellow solid 4, yield: 92%. Melting point: 167-169 °C; HRMS m/z (ESI): C₁₉H₁₇N₂O₃ [M+H⁺] theoretical value: 321.1234, measured value: 321.1240; ¹H-NMR (400MHz, CDCl₃) δ: 7.81 (m, 2H), 7.68 (m, 2H), 7.65 (m, 1H), 7.57 (m, 1H), 6.41 (d, *J* = 8.4 Hz, 1H), 5.62 (q, *J* = 6.4 Hz, 1H) , 4.27 (br, 1H), 3.62 (t, *J* = 8.4 Hz, 2H), 3.01 (t, *J* = 8.4 Hz, 2H), 1.72 (d, *J* = 6.4 Hz, 3H). ¹³C-NMR (100MHz, CDCl₃) δ: 193.45, 167.79, 156.34, 134.02, 131.96, 129.98, 129.14, 125.29, 125.01, 123.39, 107.09, 50.69, 47.12, 28.70, 15.45.

### Example 3: Preparation of Compound 5

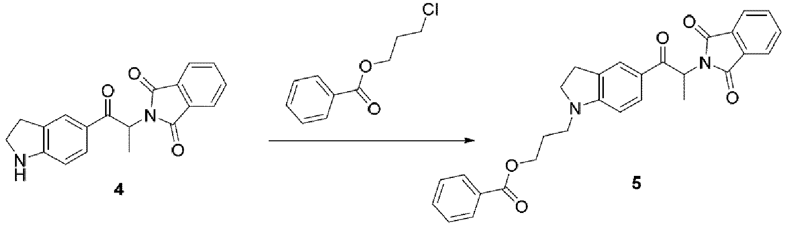

At room temperature, 32 g of compound 4 obtained from example 2, 32 g of 3-chloropropyl benzoate, 26 g of potassium iodide, 33 g of potassium carbonate, 2.2 g of tetrabutylammonium bromide and 600 mL of DMF were fed into the reaction vessel, wherein the molar ratio of compound 4, 3-chloropropyl benzoate, potassium carbonate, potassium iodide, phase transfer catalyst tetrabutylammonium bromide is 1:1.6:2.4:1.6:0.07.

Then the temperature was slowly increased to 90-100 °C to carry out the reaction, liquid chromatograph was used to monitor the reaction until it was completed (18-26 hours), the reaction solution was distilled to remove DMF under reduced pressure, the obtained residues were slowly added to a mixture of dichloromethane (400mL) and 5% hydrochloric acid aqueous solution (800mL), and the mixture layered after being stirred. The organic phase was washed successively with saturated sodium bicarbonate solution and saturated salt solution, the organic layer was collected then was dried over anhydrous sodium sulfate, then was filtered, and the filtrate was concentrated to dryness under reduced pressure to obtain 39.5g of yellow liquid 5, yield: 82%. ESI-HRMS (m/z): C₂₉H₂₇N₂O₅[M+H⁺] theoretical value: 483.1914, measured value: 483.1919; ¹H-NMR (400MHz, CDCl₃) δ: 8.01 (m, 2H), 7.81 (m, 2H), 7.69 (m, 2H), 7.61-7.43 (m, 5H), 6.30 (d, *J* = 6.4 Hz, 1H), 5.62 (q, *J* = 6.4 Hz, 1H), 4.39 (t, *J* = 8.4 Hz, 2H), 3.55 (t, *J* = 8.4 Hz, 2H), 3.32 (t, *J* = 8.4 Hz, 2H), 2.99 (d, *J* = 8.4 Hz, 2H), 2.05 (m, 2H), 3.32 (d, *J* = 6.4 Hz, 3H); ¹³C-NMR (100 MHz, CDCl₃) δ: 192.99, 167.80, 166.47, 156.17, 133.99, 133.12, 132.01, 130.45, 130.06, 129.83, 129.51, 128.47, 124.97, 123.90, 123.38, 104.31, 62.50, 52.43, 50. 63, 44.33, 27.56, 26.58, 15.51.

### Example 4: Preparation of Compound 6

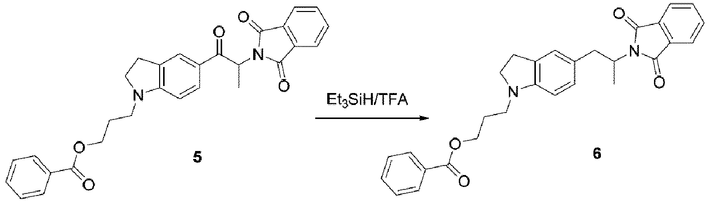

At -5 to 0 °C, 30 g of trifluoroacetic acid, 48.2 g of compound 5 obtained from example 3 and 300 mL of dichloromethane were fed into the reaction vessel in which an organic solvent was filled, and 24 g of trichlorosilane in 100 mL of dichlorosilane was slowly added dropwise at this temperature. The molar ratio of compound 5, triethylsilane and trifluoroacetic acid is 1:2.0:2.6.

After the completion of the dropwise addition, the reaction was carried out for 1 hour while keeping the temperature, and then the temperature was slowly increased to room temperature to carry out the reaction, liquid chromatograph was used to monitor the reaction until it was completed (8-12 hours), the reaction solution was poured into ice water, the organic phase was washed successively with sodium bicarbonate solution (800mL) and saturated salt solution (800mL), the organic layer was collected then was dried over anhydrous sodium sulfate, then was filtered, and the filtrate was concentrated to dryness under reduced pressure to obtain 44.4 g of pale yellow solid 6, yield: 95%. Melting point: 80-82 °C; ESI-HRMS (m/z): C₂₉H₂₉N₂O₄ [M+H⁺] theoretical value: 469.2122, measured value: 469.2117; ¹H-NMR (400 MHz, CDCl₃) δ: 8.05 (m, 2H), 7.78 (m, 2H), 7.65 (m, 2H), 7.58 (m, 1H), 7.44 (m, 2H), 6.92 (s, 1H), 6.87 (d, *J* = 8.0 Hz , 1H), 6.32 (d, *J* = 8.0 Hz, 1H), 4.58 (m, 1H), 4.41 (t, *J* = 6.4 Hz, 2H), 3.32 (m, 2H), 3.15 (m, 3H), 3.00 (m, 1H), 2.85 (m, 2H), 2.05 (m, 2H), 1.47 (d*, J* = 6.4 Hz, 3H); ¹³C-NMR (100 MHz, CDCl₃) δ: 168.48, 166.57, 151.11 , 133.69, 132.97, 132.00, 130.33, 130.30, 129.55, 128.41, 127.79, 127.77, 125.19, 122.98, 106.85, 62.93, 53.51, 49.00, 46.45, 39.41, 28.53, 26.92, 18.07.

### Example 5: Preparation of Compound 7

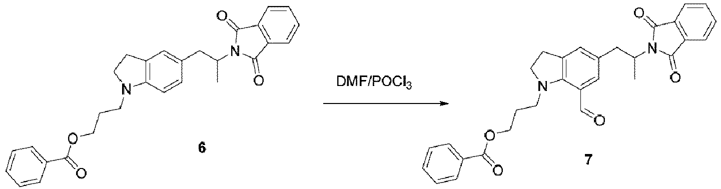

Under nitrogen protection, 30 g of phosphorus oxychloride was slowly added dropwise to a DMF (45mL) solution of 46.8 g of compound 6 obtained from example 4 at -5 to 0°C, optionally, the molar ratio of compound 6 and phosphorus oxychloride is 1: (2.0-3.0), preferably 1:2.0. Then, the reaction was carried out by increasing the temperature to room temperature under stirring, liquid chromatograph was used to monitor the reaction until it was completed (18-24 hours), the reaction solution was concentrated to dryness, the residues were poured into 500 mL of ice water, then were extracted with 300 mL of dichloromethane, the organic layer was washed successively with 800 mL of sodium bicarbonate solution and 800 mL of saturated salt solution, the organic layer was collected then was dried over anhydrous sodium sulfate, then was filtered, and the filtrate was concentrated to dryness under reduced pressure to obtain 45.1 g of yellow solid 7, yield: 91%. Melting point: 146-148 °C; ESI-HRMS (m/z): C₃₀H₂₉N₂O₅ [M+H⁺] theoretical value: 497.2071, measured value: 497.2065; ¹H-NMR (400 MHz, CDCl₃)δ: 9.79 (s, 1H), 8.03 (m, 2H), 7.76 (m, 2H), 7.66 (m, 2H), 7.57 (m, 1H), 7.46 (m, 2H), 7.18 (s, 1H), 7.03 (s, 1H), 4.58 (m, 1H), 4.37 (t, *J* = 6.4 Hz, 2H), 3.60 (m, 4H), 3.21 (m, 1H), 3.00 (m, 3H), 2.05 (m, 2H), 1.50 (d, *J* = 6.4 Hz, 3H); ¹³C-NMR (100 MHz, CDCl₃) δ: 189.84, 168.41, 166.55, 151.58, 133.84, 133.72, 132.98, 131.97, 131.83, 130.17, 130.09, 129.61, 128.41, 127.06, 123.04, 118.19, 62.80, 54.61, 49.86, 48.54, 38.82, 27.77, 26.76, 18.14.

### Example 6: Preparation of Compound 8

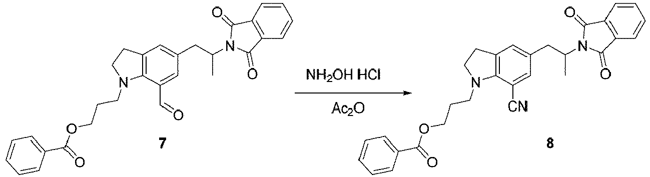

49.6 g of compound 7 obtained from example 5 was dissolved in 500 mL of THF, 13.8 g of potassium carbonate and 7.0 g of hydroxylamine hydrochloride were added. Optionally, the molar ratio of compound 7, hydroxylamine hydrochloride and potassium carbonate was 1: (1.0-2.0):(1.0-2.0), preferably 1:1.0:1.0. The temperature was slowly increased to 50 °C and the mixture was stirred for 1 hour, then 25 mL of acetic anhydride was slowly added dropwise, followed by heating the reaction liquid to carry out reflux reaction. Liquid chromatograph was used to monitor the reaction until it was completed (18-24 hours), the synthetic liquid was filtered after the termination of the reaction, the filtrate was concentrated to dryness, 500 mL of water was added to the residues, then were extracted with 800 mL of dichloromethane, the organic layer was washed successively with 800 mL of sodium bicarbonate solution and 800 mL of saturated salt solution, the organic layer was collected then was dried over anhydrous sodium sulfate, then was filtered, and the filtrate was concentrated to dryness under reduced pressure to obtain 43.9 g of white solid 8, yield: 89%. Melting point: 138-140 °C; ESI-HRMS (m/z): C₃₀H₂₈N₃O₄ [M+H⁺] theoretical value: 494.2074, measured value: 494.2079; ¹H-NMR (400 MHz, CDCl₃) δ: 8.05 (m, 2H), 7.78 (m, 2H), 7.68 (m, 2H), 7.57 (m, 1H), 7.42 (m, 2H), 6.98 (s, 1H), 6.92 (s, 1H), 4.52 (m, 1H), 4.44 (t, *J* = 6.4Hz, 2H), 3.70 (m, 2H), 3.54 (t, *J* = 8.4 Hz, 2H), 3.15 (m, 1H), 2.95 (m, 3H) , 2.11 (m, 2H), 1.49 (d, *J* = 6.4 Hz, 3H); ¹³C-NMR (100MHz, CDCl₃) δ: 168.38, 166.59, 151.78, 133.91, 132.98, 132.77, 131.81, 131.39, 130.12, 129.66, 129.16, 128.36, 127.11, 123.12, 119.27, 87.84, 62.55, 53.34, 48.46, 45.21, 38.68, 27.28, 27.13, 18.15.

### Example 7: Preparation of Compound 9

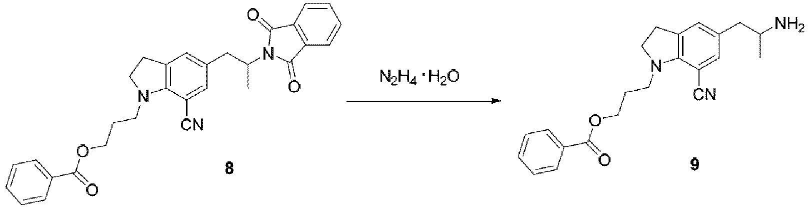

Under nitrogen protection, 30 g of hydrazine hydrate was slowly added dropwise to a THF (45 mL) solution of 49.3 g of compound 8 obtained from example 6 at -5 to 0 °C, and then the reaction was carried out by increasing the temperature until reflux under stirring, liquid chromatograph was used to monitor the reaction until it was completed (18-24 hours), the reaction liquid was concentrated to dryness, the residues were poured into 500 mL of ice water, then were extracted with 500 mL of dichloromethane, the organic layer was washed successively with 800 mL of sodium bicarbonate solution and 800 mL of saturated salt solution, the organic layer was collected then was dried over anhydrous sodium sulfate, then was filtered, and the filtrate was concentrated to dryness under reduced pressure to obtain 45.1 g of oily substance 9. ESI-HRMS (m/z): C₂₂H₂₆N₃O₂ [M+H⁺] theoretical value: 364.2020, measured value: 364.2016, the oily substance was used directly in the next step.

### Example 8: Preparation of Compound 10

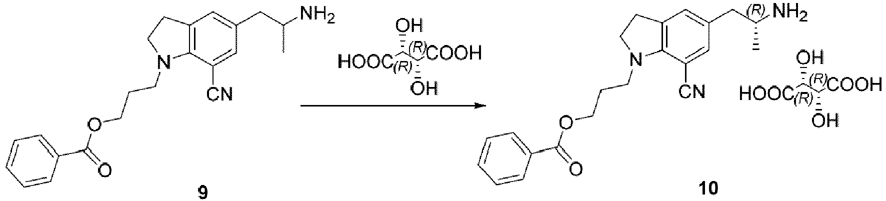

45.1 g of the oily substance (compound 9 obtained from example 7) obtained from the last step, 6.9 g of L-tartaric acid and 500 mL of tetrahydrofuran were fed into the reaction vessel, and the temperature was increased to 50-60 °C, the mixture was stirred to be dissolved, and then the temperature was slowly decreased to room temperature (25 °C) to obtain a large amount of crystals, and the obtained crystals were filtered. After drying in vacuum, 17.7 g of white crystals 10 were obtained, yield: 34%.

### Example 9: Preparation of Compound 11

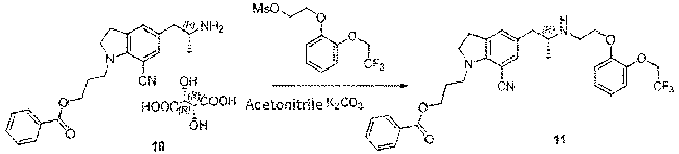

Into a reaction vessel where 27.6 g of potassium carbonate was present, 51.3 g of compound 10 obtained from example 8, 68 g of 2-[2-(2,2,2-trifluoroethoxy)phenoxy]ethyl methanesulfonate, 400 mL of acetonitrile were fed, optionally the molar ratio of compound 10, 2-[2-(2,2,2-trifluoroethoxy)phenoxy]ethyl methanesulfonate and potassium carbonate is 1:(1.0-1.5):(1.5-2.5), preferably 1:1.0:2.0.

Then, the reaction was carried out by increasing the temperature to 80-90°C under stirring, liquid chromatograph was used to monitor the reaction until it was completed (18-24 hours), the reaction liquid was concentrated to dryness, the residues were poured into 800 mL of water, then were extracted with 500 mL of ethyl acetate, the organic layer was washed with 800 mL of saturated salt solution, the organic layer was collected then was dried over anhydrous sodium sulfate, then was filtered, the filtrate was concentrated to dryness under reduced pressure to obtain 43.6 g of oily substance 11. ESI-HRMS (m/z): C₃₂H₃₅F₃N₃O₄ [M+H⁺] theoretical value: 582.2574, measured value: 582.2578, yield: 75%, the oily substance was used directly in the next step.

### Example 10: Preparation of Compound 12

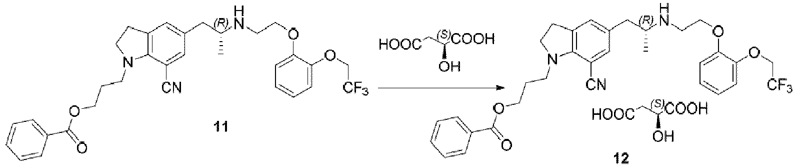

43.6 g of the compound 11 obtained from example 9, 10.1 g of L-malic acid, 250 mL of ethyl acetate and 250 mL of n-hexane were fed into a reaction vessel. Optionally, the molar ratio of compound 11 to malic acid was 1: (1.0- 1.1), preferably 1:1.05. Optionally, the volume ratio of ethyl acetate to n-hexane was 1:1.

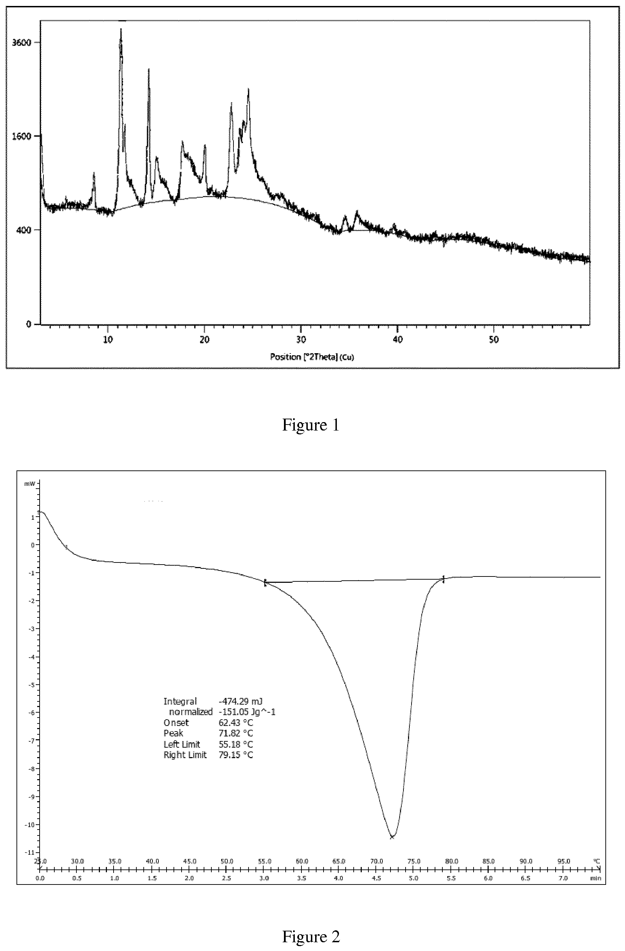

The temperature was increased to 60-70 °C and the mixture was stirred to be dissolved, and then the temperature was slowly decreased to -5 to 0 °C to crystallize, and a large amount of white solids were precipitated. The obtained solids were filtered, and dried in vacuum to give 45.6 g of white crystals 12, yield: 85%. The crystal form of the obtained compound 12 is Form I, the melting point thereof is 71-73 °C. As is shown in figure 2, a peak was found at 71.82 °C in the DSC thermogram. As is shown in figure 1, the 2θ of the main peaks of the powder X-ray diffraction pattern were 8.6°±0.2°, 11.3°±0.2°, 11.4°±0.2°, 11.8°±0.2°, 14.2°±0.2°, 15.3°±0.2°, 17.6°±0.2°, 20.1°±0.2°, 22.8°±0.2°, 23.7°±0.2°, 24.0°±0.2°, 24.6°±0.2°, 34.6°±0.2°, 35.8°±0.2°.

### Example 11: Preparation of Compound 13

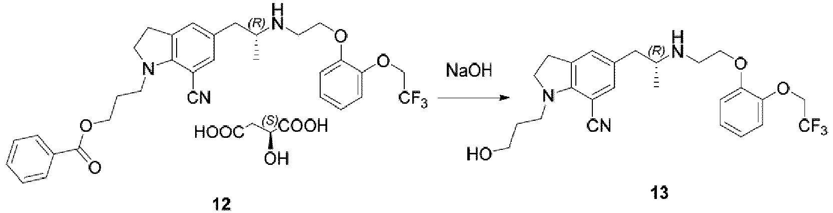

35.8 g of compound 12 obtained from example 10, 200 g of sodium hydroxide aqueous solution (10%) and 100 mL of ethanol were fed into a reaction vessel, and then the reaction was carried out at room temperature under stirring, liquid chromatograph was used to monitor the reaction until it was completed (5-10 hours), ethanol was removed from the reaction liquid, the residues were poured into 500 mL of water, then were extracted with 500 mL of dichloromethane, the organic layer was washed successively with 900 mL of sodium bicarbonate solution and 900mL of saturated salt solution, the organic layer was collected then was dried over anhydrous sodium sulfate, then was filtered, the filtrate was concentrated to dryness under reduced pressure to obtain 22.7 g of white solid 13. ESI-HRMS (m/z): C₂₅H₃₁F₃N₃O₃ [M+H⁺] theoretical value: 478.2312, measured value: 478.2307, yield: 95%.

### Example 12: Preparation of Compound 1

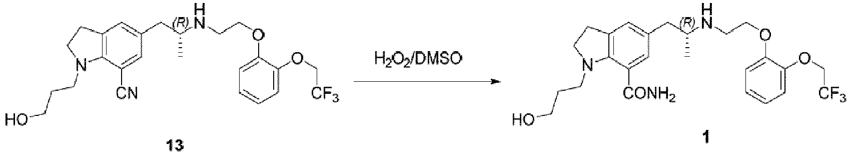

47.8 g of compound 13 obtained from example 11, 13.7 g of potassium carbonate and 500 mL of DMSO were fed into a reaction vessel, and 13.7 g of hydrogen peroxide (30%) was slowly added dropwise at 18-20 °C, optionally, the molar ratio of compound 13 to hydrogen peroxide was 1: (2.0-5.0), preferably 1:2.0.

The reaction was then carried out at the same temperature under stirring, liquid chromatograph was used to monitor the reaction until it was completed (4-8 hours), the reaction liquid was extracted with 600 mL of ethyl acetate, the organic layers were combined, and the organic layers were washed with 1 mol/L HCl aqueous solution, were washed with saturated sodium bicarbonate, dried over anhydrous sodium sulfate, concentrated under reduced pressure, and then dissolved with ethyl acetate, and then cooled naturally, crystallized, filtered, and dried to give 45.5 g of silodosin 1, ESI-HRMS (m/z): C₂₅H₃₃F₃N₃O₄ [M+H⁺] theoretical value: 496.2418, measured value: 496.2414, yield: 92%, purity >99%.

The specific examples of the present invention have been described in detail above, but they are only preferred embodiments of the present invention. From a technical point of view, based on the synthetic route of the present invention, several optimizations of the reaction conditions in the described implementation steps and the improvements of the method for obtaining the intermediates involved in the present invention should also be considered as the protection scope of the present invention, and thus the present invention is not limited to the specific examples described above.

## Claims

1. A method for synthesizing silodosin intermediate 10, **characterized in that** the method is carried out by the following reaction steps: 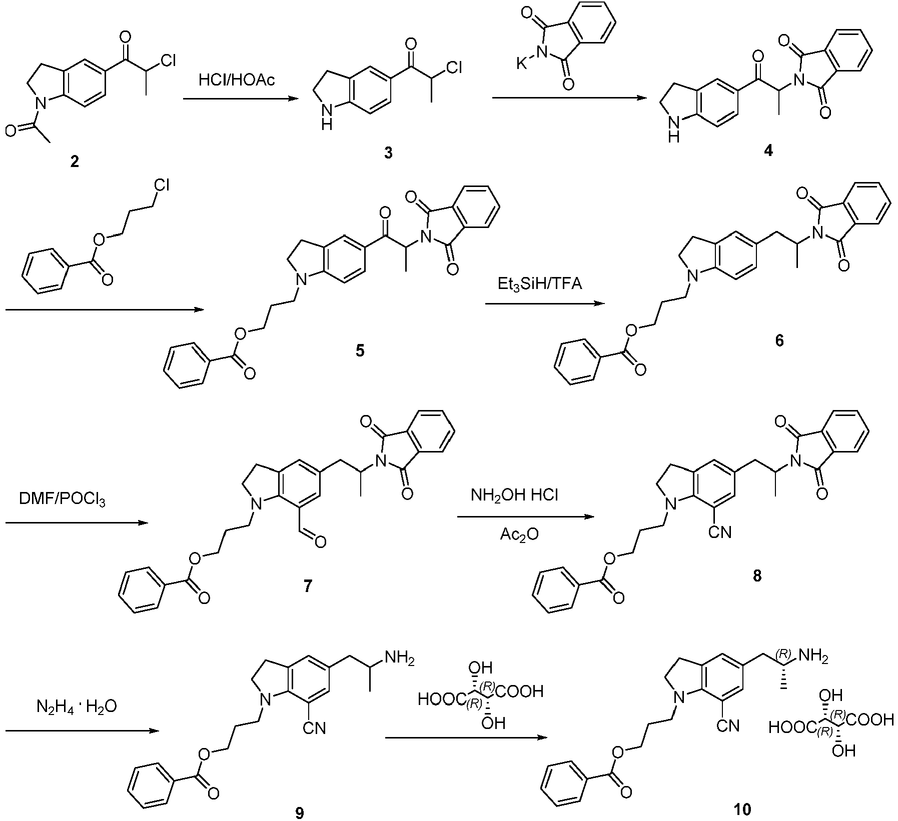 the specific steps are:
step 1, chloride 2 is deacetylated with hydrochloric acid and acetic acid to obtain indoline 3,
step 2, indoline 3 and potassium phthalimide are subjected to SN2 substitution reaction in an organic solvent under the presence of potassium carbonate to obtain imide 4.
step 3, N-alkylation reaction is carried out between imide 4 and 3-chloropropyl benzoate in an organic solvent at a certain temperature under the presence of a phase transfer catalyst, potassium carbonate and potassium iodide to obtain benzoate 5,
step 4, the carbonyl of benzoate 5 is reduced by triethylsilane and trifluoroacetic acid to obtain indoline 6,
step 5, indoline 6 is subjected to Vilsmeier reaction under the action of dimethylformamide and phosphorus oxychloride to obtain aldehyde 7,
step 6, aldehyde 7 is subjected to oximation and dehydration to obtain nitrile 8,
step 7, nitrile 8 reacts with hydrazine hydrate to remove the phthaloyl group, and the obtained amine 9 is resolved by L-tartaric acid to obtain silodosin intermediate 10.

2. The method for synthesizing silodosin intermediate 10 according to claim 1, **characterized in that**, in step 2, the molar ratio of indoline 3, potassium phthalimide and potassium carbonate is 1:(1.0-2.0):(1.0-2.0).

3. The method for synthesizing silodosin intermediate 10 according to claim 2, **characterized in that**, in step 2, the molar ratio of indoline 3, potassium phthalimide and potassium carbonate is 1:1.05:1.1.

4. The method for synthesizing silodosin intermediate 10 according to any one of claims 1-3, **characterized in that**, in step 3, the phase transfer catalyst is selected from tetrabutylammonium bromide, tetrabutylammonium chloride, tetrabutylammonium iodide, benzyltrimethylammonium chloride, benzyltriethylammonium chloride.

5. The method for synthesizing silodosin intermediate 10 according to claim 4, **characterized in that**, in step 3, the molar ratio of imide 4 to 3-chloropropyl benzoate, potassium carbonate, potassium iodide and the phase transfer catalyst is 1:(1.0-2.0):(2.0-4.0):(1.0-2.0):(0.05-0.1).

6. The method for synthesizing silodosin intermediate 10 according to claim 5, **characterized in that**, in step 3, the molar ratio of imide 4 to 3-chloropropyl benzoate, potassium carbonate, potassium iodide and the phase transfer catalyst is 1:1.6:2.4:1.6:0.07.

7. The method for synthesizing silodosin intermediate 10 according to claim 1, **characterized in that**, in step 3, the reaction temperature is 90-100 °C.

8. The method for synthesizing silodosin intermediate 10 according to claim 5 or 6, **characterized in that**, in step 3, the organic solvent is selected from dimethylformamide, dimethylacetamide, dimethyl sulfoxide, N-methyl pyrrolidone.

9. A method for synthesizing silodosin, wherein said method comprises the step of preparing the intermediate 10 according to any one of the preceding claims, and synthesizing silodosin according to the following reaction steps: in an organic solvent, a condensation reaction is carried out between the intermediate 10 and 2-[2-(2,2,2-trifluoroethoxy)phenoxy]ethyl methanesulfonate under an alkaline condition to give compound 11, the obtained compound 11 is crystallized with L-malic acid to give salt 12, salt 12 is reacted to remove the benzoyl protection under alkaline condition, and then is hydrolyzed by hydrogen peroxide under alkaline condition in an organic solvent to obtain silodosin

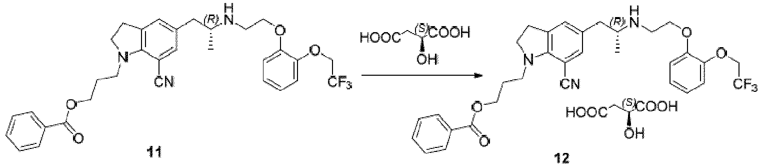

## Patentansprüche

1. Verfahren zur Synthese von Silodosin-Zwischenprodukt 10, **dadurch gekennzeichnet, dass** das Verfahren durch die folgenden Reaktionsschritte ausgeführt wird: 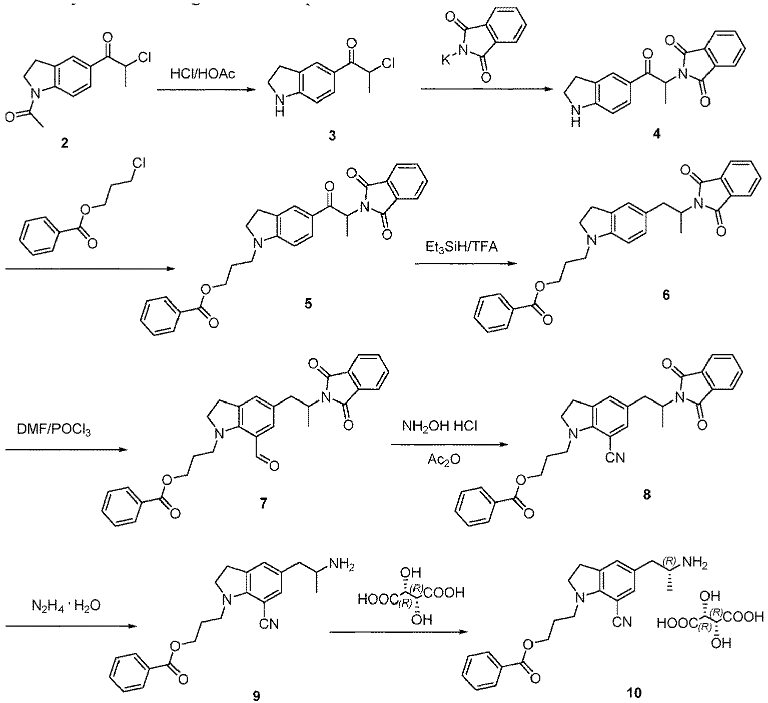 wobei die speziellen Schritte die Folgenden sind:
Schritt 1, Chlorid 2 wird mit Salzsäure und Essigsäure deacetyliert, um Indolin 3 zu erhalten,
Schritt 2, Indolin 3 und Kaliumphthalimid werden einer SN2-Substitutionsreaktion in einem organischen Lösungsmittel in Gegenwart von Kaliumkarbonat unterzogen, um Imid 4 zu erhalten,
Schritt 3, N-Alkylierungsreaktion wird zwischen Imid 4 und 3-Chlorophylbenzoat in einem organischen Lösungsmittel bei einer bestimmten Temperatur in Gegenwart von einem Phasentransfer-Katalysator, Kaliumkarbonat und Kaliumiodid ausgeführt, um Benzoat 5 zu erhalten,
Schritt 4, Benzoatcarbonyl 5 wird durch Triethylsilan und Trifluoressigsäure reduziert, um Indolin 6 zu erhalten,
Schritt 5, Indolin 6 wird einer Vilsmeier Reaktion unter der Wirkung von Dimethylformamid und Phosphoroxychlorid unterzogen, um Aldehyd 7 zu erhalten,
Schritt 6, Aldehyd 7 wird einer Oximation und Dehydrierung unterzogen, um Nitril 8 zu erhalten,
Schritt 7, Nitril 8 reagiert mit Hydrazinhydrat zum Entfernen der Phthaloyl-Gruppe, und das erhaltene Amin 9 wird durch L-Tartarsäure gelöst, um das Silodosin-Zwischenprodukt 10 zu erhalten.

2. Verfahren zur Synthese von Silodosin-Zwischenprodukt 10 nach Anspruch 1, **dadurch gekennzeichnet, dass** in Schritt 2 das Mol-Verhältnis von Indolin 3, Kaliumphthalamid und Kaliumkarbonat 1:(1,0-2,0):(1,0-2,0) ist.

3. Verfahren zur Synthese von Silodosin-Zwischenprodukt 10 nach Anspruch 2, **dadurch gekennzeichnet, dass** in Schritt 2 das Mol-Verhältnis von Indolin 3, Kaliumphthalamid und Kaliumkarbonat 1:1,05:1,1 ist.

4. Verfahren zur Synthese von Silodosin-Zwischenprodukt 10 nach einem der Ansprüche 1-3, **dadurch gekennzeichnet, dass** in Schritt 3 der Phasentransfer-Katalysator ausgewählt ist aus Tetrabutylammoniumbromid, Tetrabutylammoniumchlorid, Tetrabutylammoniumiodid, Benzyltrimethylammoniumchlorid, Benzyltriethylammoniumchlorid.

5. Verfahren zur Synthese von Silodosin-Zwischenprodukt 10 nach Anspruch 4, **dadurch gekennzeichnet, dass** in Schritt 3 das Mol-Verhältnis von Imid 4 zu 3-Chlorophylbenzoat, Kaliumkarbonat, Kaliumiodid und dem Phasentransfer-Katalysator 1:(1,0-2,0):(2,0-4,0): (1,0-2,0) : (0,05-0,1) ist.

6. Verfahren zur Synthese von Silodosin-Zwischenprodukt 10 nach Anspruch 5, **dadurch gekennzeichnet, dass** in Schritt 3 das Mol-Verhältnis von Imid 4 zu 3-Chlorophylbenzoat, Kaliumkarbonat, Kaliumiodid und dem Phasentransfer-Katalysator 1:1,6:2,4:1,6:0,07 ist.

7. Verfahren zur Synthese von Silodosin-Zwischenprodukt 10 nach Anspruch 1, **dadurch gekennzeichnet, dass** in Schritt 3 die Reaktionstemperatur 90-100°C beträgt.

8. Verfahren zur Synthese von Silodosin-Zwischenprodukt 10 nach Anspruch 5 oder 6, **dadurch gekennzeichnet, dass** in Schritt 3 das organische Lösungsmittel ausgewählt ist aus Dimethylformamid, Dimetylacetamid, Dimethylsulfoxid, N-Methylpyrrolidon.

9. Verfahren zur Synthese von Silodosin, wobei das Verfahren den Schritt des Herstellens des Zwischenprodukts 10 nach einem der vorstehenden Ansprüche und die Synthese von Silodosin nach den folgenden Reaktionsschritten umfasst: in einem organischen Lösungsmittel wird eine Kondensationsreaktion zwischen dem Zwischenprodukt 10 und 2-[2-(2,2,2-Trifluorethoxy)phenoxy]ethylmethansulfat unter einer alkalischen Bedingung ausgeführt, um eine Verbindung 11 zu ergeben, wobei die erhaltene Verbindung 11 mit L-Malinsäure kristallisiert wird, um ein Salz 12 zu ergeben, Salz 12 reagiert wird, um den Benzoyl-Schutz unter einer alkalischen Bedingung zu entfernen, und danach durch Wasserstoffperoxid unter einer alkalischen Bedingung in einem organischen Lösungsmittel hydrolisiert wird, um Silodosin zu erhalten

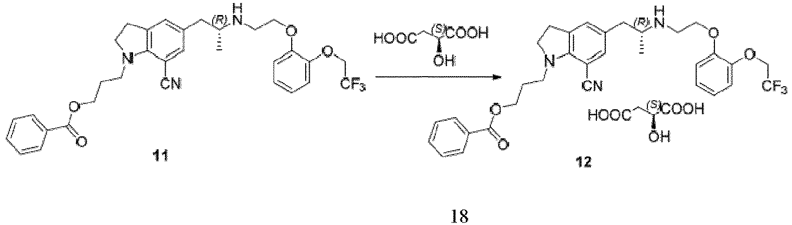

## Revendications

1. Procédé de synthèse de l'intermédiaire 10 de silodosine, **caractérisé en ce que** le procédé est mis en œuvre par les étapes réactionnelles suivantes : 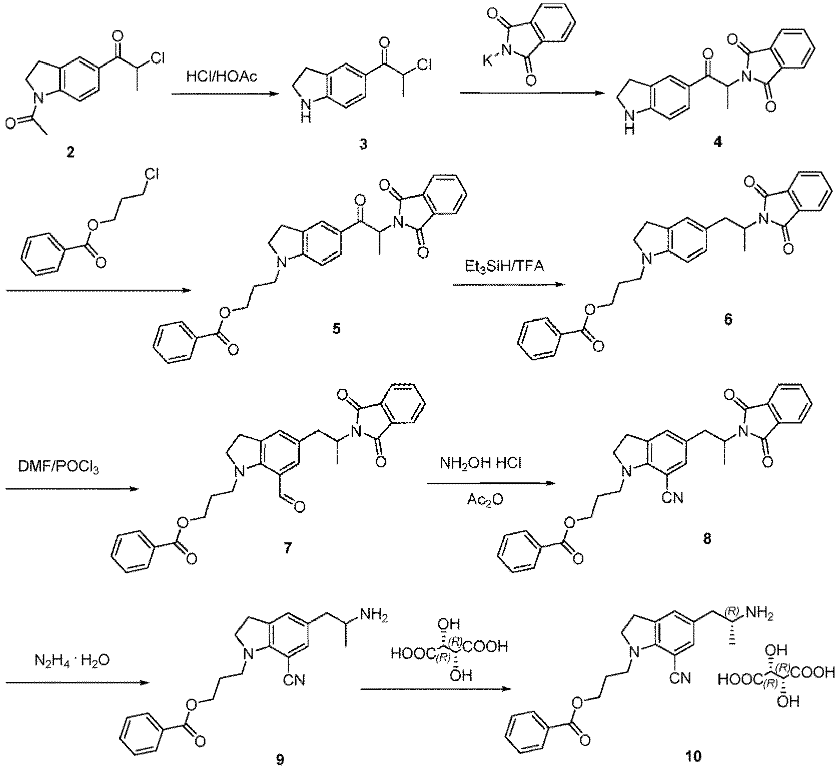 les étapes spécifiques sont :
étape 1, du chlorure 2 est désacétylé avec de l'acide chlorhydrique et de l'acide acétique pour obtenir de l'indoline 3,
étape 2, l'indoline 3 et du phtalimide de potassium sont soumis à une réaction de substitution SN2 dans un solvant organique en présence de carbonate de potassium pour obtenir de l'imide 4,
étape 3, une réaction de N-alkylation est mise en œuvre entre l'imide 4 et du benzoate de 3-chloropropyle dans un solvant organique à une certaine température en présence d'un catalyseur de transfert de phase, de carbonate de potassium et d'iodure de potassium pour obtenir du benzoate 5,
étape 4, le carbonyle de benzoate 5 est réduit par du triéthylsilane et de l'acide trifluoroacétique pour obtenir de l'indoline 6,
étape 5, l'indoline 6 est soumise à une réaction de Vilsmeier sous l'action de diméthylformamide et d'oxychlorure de phosphore pour obtenir de l'aldéhyde 7,
étape 6, l'aldéhyde 7 est soumis à une oximation et à une déshydratation pour obtenir du nitrile 8,
étape 7, le nitrile 8 réagit avec de l'hydrate d'hydrazine pour extraire un groupe phtaloyle et l'amine 9 obtenue est transformée par de l'acide L-tartarique pour obtenir l'intermédiaire 10 de silodosine.

2. Procédé de synthèse de l'intermédiaire 10 de silodosine selon la revendication 1, **caractérisé en ce que**, à l'étape 2, le rapport molaire d'indoline 3, phtalimide de potassium et carbonate de potassium est 1 :(1,0-2,0):(1,0-2,0).

3. Procédé de synthèse de l'intermédiaire 10 de silodosine selon la revendication 2, **caractérisé en ce que**, à l'étape 2, le rapport molaire d'indoline 3, phtalimide de potassium et carbonate de potassium est 1 :1,05 :1,1.

4. Procédé de synthèse de l'intermédiaire 10 de silodosine selon l'une quelconque des revendications 1-3, **caractérisé en ce que**, à l'étape 3, le catalyseur de transfert de phase est sélectionné parmi le bromure de tétrabutylammonium, le chlorure de tétrabutylammonium, l'iodure de tétrabutylammonium, le chlorure de benzyltriméthylammonium, le chlorure de benzyltriéthylammonium.

5. Procédé de synthèse de l'intermédiaire 10 de silodosine selon la revendication 4, **caractérisé en ce que**, à l'étape 3, le rapport molaire entre l'imide 4 et le benzoate de 3-chloropropyle, le carbonate de potassium, l'iodure de potassium et le catalyseur de transfert de phase est 1:(1,0-2,0):(2,0-4,0):(1,0-2,0):(0,05-0,1).

6. Procédé de synthèse de l'intermédiaire 10 de silodosine selon la revendication 5, **caractérisé en ce que**, à l'étape 3, le rapport molaire entre l'imide 4 et le benzoate de 3-chloropropyle, le carbonate de potassium, l'iodure de potassium et le catalyseur de transfert de phase est 1:1,6:2,4:1,6:0,07.

7. Procédé de synthèse de l'intermédiaire 10 de silodosine selon la revendication 1, **caractérisé en ce que**, à l'étape 3, la température réactionnelle est 90-100 °C.

8. Procédé de synthèse de l'intermédiaire 10 de silodosine selon la revendication 5 ou 6, **caractérisé en ce que**, à l'étape 3, le solvant organique est sélectionné parmi le diméthylformamide, le diméthylacétamide, le diméthylsulfoxyde, la N-méthylpyrrolidone.

9. Procédé de synthèse de silodosine, dans lequel le procédé comprend l'étape de préparation de l'intermédiaire 10 selon l'une quelconque des revendications précédentes, et la synthèse de silodosine selon les étapes réactionnelles suivantes : dans un solvant organique, une réaction de condensation est mise en œuvre entre l'intermédiaire 10 et du méthanesulfonate de 2-[2-(2,2,2-trifluoroéthoxy)phénoxy]éthyle dans des conditions alcalines pour donner un composé 11, le composé 11 obtenu est cristallisé avec de l'acide L-malique pour donner un sel 12, le sel 12 est mis à réagir pour éliminer la protection benzoyle dans des conditions alcalines, puis est hydrolysé par du peroxyde d'hydrogène dans des conditions alcalines dans un solvant organique pour obtenir la silodosine
